# EUROPÄISCHE PATENTSCHRIFT

(11) **EP 2 946 318 B1**
(45) Veröffentlichungstag und Bekanntmachung des Hinweises auf die Patenterteilung: **19.12.2018**
(21) Anmeldenummer: 14704293.1
(22) Anmeldetag: 16.01.2014
(51) Int. Cl.: G06F 19/00, H04M 1/725, G16H 40/63, H04W 4/80

(54) **VERFAHREN UND VORRICHTUNG ZUR DRAHTLOSEN STEUERUNG EINES MEDIZINISCHEN GERÄTES**
METHOD AND DEVICE FOR WIRELESSLY CONTROLLING A MEDICAL DEVICE
PROCÉDÉ ET DISPOSITIF DE COMMANDE SANS FIL D'UN APPAREIL MÉDICAL

(30) Priorität: 16.01.2013 DE 102013100428
(43) Veröffentlichungstag der Anmeldung: 25.11.2015
(73) Patentinhaber: Maquet GmbH, 76437 Rastatt (DE)
(72) Erfinder: MANKOPF, Michael, 91096 Möhrendorf (DE); FRÜH, Michael, 77855 Achern (DE); RUCH, Jürgen, 77652 Offenburg (DE)
(74) Vertreter: Zacco GmbH
(86) Internationale Anmeldenummer: PCT/EP2014/050801
(87) Internationale Veröffentlichungsnummer: WO 2014/111468

(56) Entgegenhaltungen:
- WO-A1-2006/052801
- WO-A1-2007/041843
- GB-A- 2 475 091
- US-A1- 2003 093 503
- US-A1- 2008 125 064
- US-A1- 2009 080 348

## Beschreibung

Die Erfindung betrifft ein Verfahren und eine Vorrichtung zum drahtlosen Steuern eines medizinischen Gerätes mit Hilfe einer Fernbedienung. Zum Bedienen von medizinischen Geräten, wie Operationstischen, sind eine Vielzahl von Lösungen, wie sie beispielsweise in den Dokumenten DE 10 2005 054 230 A1, DE 10 2007 060 810 A1, DE 10 2007 060 808 A1, DE 10 2007 060 811 A1 und DE 10 2005 054 223 A1 offenbart sind, bekannt. Ferner sind aus dem Bereich der Unterhaltungselektronik eine Vielzahl von programmierbaren und lernbaren Fernbedienungen bekannt, die jedoch den hohen Anforderungen in Bezug auf die Fehlersicherheit an Fernbedienungen für medizinische Geräte nicht gerecht werden.

Die US2008/125064 A1 offenbart ein Verfahren gemäß dem Oberbegriff des Anspruchs 1. Weiterer Stand der Technik ist aus der US 2003/093503 A1 bekannt.

Ausgehend von dem bekannten Stand der Technik liegt die Aufgabe der Erfindung darin, ein Verfahren und eine Anordnung zum drahtlosen Steuern eines medizinischen Gerätes mit Hilfe einer Fernbedienung anzugeben, durch die eine komfortable und sichere Bedienung des medizinischen Gerätes möglich ist.

Diese Aufgabe wird durch ein Verfahren zum drahtlosen Steuern eines medizinischen Gerätes mit den Merkmalen des Anspruchs 1 sowie durch eine Anordnung mit den Merkmalen des unabhängigen Anordnungsanspruchs gelöst. Vorteilhafte Weiterbildungen der Erfindung sind in den abhängigen Ansprüchen angegeben.

Durch die Erfindung kann ein einfaches elektrisches Gerät, das einen Touchscreen aufweist und mit einer weiteren Eingabeeinheit gekoppelt wird, als Fernbedienung zum Steuern eines medizinischen Gerätes genutzt werden. Ein solches einfaches elektrisches Gerät kann ein Smartphone, ein Medienplayer oder ein Tablet-Computer sein, insbesondere ein iPod, ein iPhone oder ein iPad der Firma Apple Computer Incorporation.

Erfindungsgemäß wird die Steuerfunktion nur dann ausgeführt, wenn mit Hilfe der ersten Eingabeeinheit eine Steuerinformation eingegeben und über die von der ersten Eingabeeinheit unabhängige zweite Eingabeeinheit eine Freigabeinformation eingegeben worden ist. Dadurch führen versehentliche Eingaben über die erste Eingabeeinheit nicht unmittelbar zu der Steuerfunktion, sodass die versehentliche Steuerung des medizinischen Gerätes vermieden wird. Insbesondere bei einer versehentlichen Bedienung eines bei der Behandlung eines Patienten oder bei der Operation eines Patienten eingesetzten medizinischen Gerätes hätte eine versehentliche Bedienung fatale Folgen für die Gesundheit und das Leben des Patienten. Zumindest die erste und die zweite Eingabeeinheit sind Bestandteile der Fernbedienung zur drahtlosen Steuerung des medizinischen Gerätes.

Bei einer vorteilhaften Weiterbildung wird die Steuerinformation nur von der Fernbedienung zum Gerät gesendet, wenn die Steuerinformation über die erste Eingabeeinheit und die Freigabeinformation über die zweite Eingabeeinheit gleichzeitig oder innerhalb eines voreingestellten Zeitfenster eingegeben werden. Dadurch wird erreicht, dass ein enger zeitlicher Zusammenhang zwischen der Eingabe der Steuerinformation und der Eingabe der Freigabeinformation stehen muss, sodass Fehlbedienungen wirksam vermieden werden.

Ferner ist es vorteilhaft, wenn Daten mit den Steuerinformationen von einer ersten Steuereinheit der ersten Eingabeeinheit zu einer zweiten Steuereinheit der zweiten Eingabeeinheit übertragen werden, und wenn die Steuerinformation von der zweiten Steuereinheit nur dann weiter zum zu steuernden medizinischen Gerät übertragen wird, wenn durch die Steuereinheit die Freigabeinformation gleichzeitig oder innerhalb eines voreingestellten Zeitfensters detektiert wird. Durch das Vorsehen der zweiten Steuereinheit kann auf einfache Art und Weise überwacht werden, ob die Freigabeinformation gleichzeitig oder innerhalb eines voreingestellten Zeitfensters nach der Übertragung der Steuerinformation von der ersten Steuereinheit zur zweiten Steuereinheit vorliegt. Dadurch kann auf einfache Art und Weise die erfindungsgemäße Abhängigkeit des Sendens der Steuerinformation vom Vorliegen der Freigabeinformation realisiert werden, ohne dass bauliche Veränderungen der ersten Eingabeeinheit oder des zu steuernden medizinischen Gerätes erforderlich sind. Dies ist insbesondere dann besonders vorteilhaft, wenn als erste Eingabeeinheit ein einfaches elektrisches Gerät, das einen Touchscreen aufweist, genutzt wird. Dadurch ist eine einfache kostengünstige Realisierung einer Fernbedienung zum drahtlosen Steuern eines medizinischen Gerätes möglich. Vorzugsweise dient das einfache elektrische Gerät in Kombination mit der zweiten Eingabeeinheit als Fernbedienung. Das einfache elektrische Gerät wird vorzugsweise zumindest teilweise in ein Gehäuse aufgenommen, in dem auch die zweite Eingabeeinheit aufgenommen ist, wobei die Anordnung aus dem einfachen elektrischen Gerät, der zweiten Eingabeeinheit und dem Gehäuse die Fernbedienung zur drahtlosen Steuerung des medizinischen Gerätes bilden. Das Zeitfenster umfasst vorzugsweise einen Bereich von 2 Sekunden vor der Eingabe der Steuerinformation bis 2 Sekunden nach der Eingabe der Steuerinformation, vorzugsweise 1 Sekunde vor der Eingabe der Steuerinformation bis 1 Sekunde nach der Eingabe der Steuerinformation, insbesondere 0,5 Sekunde vor der Eingabe der Steuerinformation bis 0,5 Sekunde nach der Eingabe der Steuerinformation.

Alternativ oder zusätzlich wird die Steuerfunktion des medizinischen Gerätes durch eine Steuereinheit des Gerätes nur dann aktiviert und/oder deaktiviert, wenn die Steuerinformation und die Freigabeinformation innerhalb eines voreingestellten Zeitfensters oder gleichzeitig von der Fernbedienung zum Gerät übertragen werden. Hierdurch kann alternativ oder zusätzlich eine Überprüfung des Vorliegens der Freigabeinformation im zu steuernden medizinischen Gerät selber erfolgen. Dadurch wird ein hohes Maß an Sicherheit erreicht. Das Zeitfenster umfasst vorzugsweise einen Bereich von 2 Sekunden vor der Eingabe der Steuerinformation bis 2 Sekunden nach der Eingabe der Steuerinformation, vorzugsweise 1 Sekunde vor der Eingabe der Steuerinformation bis 1 Sekunde nach der Eingabe der Steuerinformation, insbesondere 0,5 Sekunde vor der Eingabe der Steuerinformation bis 0,5 Sekunde nach der Eingabe der Steuerinformation.

Erfindungsgemäß wird die zweite Eingabeeinheit durch mindestens einen Schalttransponder bereitgestellt, der einen Transponder und einen mit diesem verbundenes Betätigungselement umfasst. Das Betätigungselement kann beispielsweise ein Taster sein. Als Transponder kann ein aktiver Transponder oder ein passiver Transponder verwendet werden. Aktive Transponder verfügen über eine eigene Energieversorgung. Diese Energieversorgung kann über eine der Fernbedienung angeordnete Batterie oder einen dort angeordneten Akkumulator erfolgen. Alternativ oder zusätzlich kann die Energieversorgung des aktiven Transponders auch durch eine Batterie oder einen Akkumulator der ersten Eingabeeinheit mit Energie versorgt werden. Aktive Transponder haben im Allgemeinen eine größere Reichweite als passive Transponder. Passive Transponder beziehen die zur Kommunikation und zur Abarbeitung interner Prozesse benötigte Energie ausschließlich aus dem Feld einer Schreib-/Leseeinheit, sodass die passiven Transponder keine eigene Stromversorgung benötigen, wobei die Kommunikationsreichweite von passiven Transpondern im Allgemeinen geringer ist als von aktiven Transpondern. Jedoch sind im Stand der Technik Möglichkeiten bekannt, auch die Reichweite von passiven Transpondern zu erhöhen. Eine Vorrichtung und ein Verfahren zur drahtlosen Energie- und/oder Datenübertragung zwischen einem Quellgerät und mindestens einem Zielgerät sind beispielsweise aus dem Dokument DE 10 2007 060 811 A1 bekannt.

Ferner ist es vorteilhaft, wenn der Schalttransponder zumindest in einem Schaltzustand des Betätigungselements mit einer Kommunikationseinheit, vorzugsweise mit einer Leseeinheit kommuniziert, wobei die Kommunikationseinheit mit einer Steuereinheit des Gerätes zumindest über eine Datenleitung verbunden ist. Dadurch ist eine einfache Kommunikation des Schalttransponders mit der Steuereinheit des medizinischen Gerätes über die Kommunikationseinheit möglich. Die Datenleitung zwischen der Kommunikationseinheit und dem medizinischen Gerät kann als drahtgebundene Datenleitung oder als drahtlose Datenleitung ausgeführt sein. Ferner kann die Kommunikationseinheit in das zu steuernde medizinische Gerät integriert sein.

Besonders vorteilhaft ist es, wenn der Transponder ein passiver, halb-aktiver oder aktiver RFID-Tag ist. Dadurch kann eine einfache und kostengünstige Kommunikation zwischen der zweiten Eingabeeinheit und dem zu steuernden medizinischen Gerätes und/oder der ersten Eingabeeinheit erfolgen.

Ferner ist es vorteilhaft, wenn die Energieversorgung des Transponders zumindest mit der zur Kommunikation mit einer Kommunikationseinheit erforderlichen Energie jeweils über eine drahtlose Kopplung mit einer Energieversorgungseinheit erfolgt, wobei die Energieversorgungseinheit vorzugsweiser Bestandteil der Kommunikationseinheit ist. Dadurch kann der Transponder die zu seinem Betrieb erforderliche Energie einfach aus dem Feld einer als Schreib-/Leseeinheit ausgeführten Kommunikationseinheit erhalten.

Ferner ist es vorteilhaft, wenn die drahtlose Kommunikation zwischen dem Transponder und einer Kommunikationseinheit im UHF-Übertragungsband erfolgt. Dadurch ist eine einfache und sichere Datenübertragung möglich.

Ferner ist es vorteilhaft, wenn das Betätigungselement der zweiten Eingabeeinheit den Empfangskreis des Transponders in einer ersten Schaltstellung des Betätigungselements kurzschließt, wobei die Energieversorgungseinheit zumindest die zur Kommunikation mit mindestens einer Kommunikationseinheit von dem Transponder benötigte Energie elektrisch und/oder elektromagnetisch zum Empfangskreis überträgt. In einer zweiten Schaltstellung des Betätigungselements kann der Transponder eine Information an die Kommunikationseinheit übertragen, insbesondere eine ID des Transponders, die von der Kommunikationseinheit ausgewertet und als Freigabeinformation interpretiert wird.

Besonders vorteilhaft ist es, wenn die zweite Eingabeeinheit keine austauschbare Batterie und keinen aufladbaren Akkumulator enthält. Dies kann insbesondere dadurch erreicht werden, dass die Stromversorgung der zweiten Eingabeeinheit über die Stromversorgung der ersten Eingabeeinheit erfolgt oder die zu ihrem Betrieb erforderliche elektrische Energie aus einem von einer als Schreib-/Leseeinheit ausgeführten Kommunikationseinheit bereitgestellten elektrischen bzw. elektromagnetischen Feld bezieht.

Besonders vorteilhaft ist es, wenn die zweite Eingabeeinheit ein Gehäuse mit einem Aufnahmefach hat, in dem die erste Eingabeeinheit derart aufgenommen wird, dass der Touchscreen der ersten Eingabeeinheit bedienbar ist. Dadurch kann eine einfache Baueinheit aus erster Eingabeeinheit und zweiter Eingabeeinheit erzeugt werden, die von einer Bedienperson einfach als Gesamtanordnung gehandhabt werden kann. Besonders vorteilhaft ist es, wenn die erste Eingabeeinheit in das Aufnahmefach eingeschoben wird. Dadurch kann die erste Eingabeeinheit einfach und sicher im Gehäuse gehalten werden. Ferner ist es dann auch einfach möglich, eine elektrische Steckverbindung zwischen dem Gehäuse und der ersten Eingabeeinheit herzustellen.

Über einen solchen Steckverbinder kann eine Stromversorgung der ersten Eingabeeinheit erfolgen und/oder Daten zwischen einer Steuereinheit der ersten Eingabeeinheit und einem Gehäuse angeordneten zweiten Steuereinheit übertragen werden. Alternativ oder zusätzlich kann über den Steckverbinder auch eine Stromversorgung der zweiten Eingabeeinheit bzw. der im Gehäuse angeordneten zweiten Steuereinheit erfolgen. Dadurch ist eine besonders einfache Stromversorgung und/oder Kopplung der ersten Eingabeeinheit und der zweiten Eingabeeinheit möglich. Besonders vorteilhaft ist es, wenn die Freigabeinformation über ein von der Außenseite des Gehäuses zugängliches außerhalb des Aufnahmebereichs angeordnetes Betätigungselement der zweiten Eingabeeinheit, eingegeben wird. Dadurch ist eine einfache Bedienung möglich.

Besonders vorteilhaft ist es, wenn mit Hilfe der Steuerfunktion ein Antrieb des zu steuernden medizinischen Gerätes aktiviert und/oder deaktiviert wird, insbesondere einen Stellantrieb und/oder ein Antrieb für ein Verriegelungselement. Dadurch können auf einfache Art und Weise eine Vielzahl von Steuerfunktionen des medizinischen Gerätes einfach durch die Fernbedienung bereitgestellt werden.

Erfindungsgemäß ist das medizinische Gerät ein Operationstisch. Dann kann mit Hilfe der Fernbedienung einfach eine Bedienfunktion insbesondere während der Operation eines Patienten, ausgeführt werden.

Besonders vorteilhaft ist es, wenn die Daten mit der Steuerinformation und/oder der Freigabeinformation von der ersten Eingabeeinheit, von der zweiten Eingabeeinheit und/oder von der zweiten Steuereinheit verschlüsselt zum zu steuernden medizinischen Gerät übertragen werden. Insbesondere können diese Daten mehrfach symmetrisch kryptografisch verschlüsselt übertragen werden. Dadurch können Manipulationen und versehentliche Fehlbedienungen vermieden werden, da sowohl Daten mit anderer kryptografischer Verschlüsselung von der Steuereinheit des medizinischen Gerätes nicht verarbeitet werden können, sodass versehentliche Fehlbedienungen verhindert werden, als auch böswillige Manipulationen zum Schaden des Anwenders und/oder Patienten wirksam vermieden werden.

Die gemäß einem zweiten Aspekt der Erfindung beanspruchte Vorrichtung zur drahtlosen Steuerung eines medizinischen Gerätes kann insbesondere dadurch weitergebildet werden, dass die erste Eingabeeinheit ein über dem Touchscreen bereitgestelltes Tastenfeld zur Eingabe der Steuerinformation hat und dass die zweite Eingabeeinheit ein Betätigungselement zur Eingabe der Freigabeinformation hat. Dadurch ist eine einfache und komfortable Eingabe der Steuerinformation und der Freigabeinformation möglich.

Ferner ist es vorteilhaft, im Gehäuse zumindest eine Steuereinheit anzuordnen, die veranlasst, dass die Steuerinformation beim gleichzeitigen oder innerhalb eines voreingestellten Zeitfensters vor und/oder nach der Eingabe der Steuerinformation Vorliegen der Freigabeinformation zum Gerät gesendet wird. Dadurch kann die Fehlersicherheit erhöht und Fehlereingaben können vermieden werden. Das Zeitfenster umfasst vorzugsweise einen Bereich von 2 Sekunden vor der Eingabe der Steuerinformation bis 2 Sekunden nach der Eingabe der Steuerinformation, vorzugsweise 1 Sekunde vor der Eingabe der Steuerinformation bis 1 Sekunde nach der Eingabe der Steuerinformation, insbesondere 0,5 Sekunde vor der Eingabe der Steuerinformation bis 0,5 Sekunde nach der Eingabe der Steuerinformation.

Ferner ist es vorteilhaft, im oder am Gehäuse eine Steuereinheit, vorzugsweise einen Transponder, anzuordnen. Die Steuereinheit bzw. der Transponder senden beim Betätigen des Betätigungselements der zweiten Eingabeeinheit ein Freigabesignal zum zu bedienenden Gerät oder stoppen das Senden eines Blockierungssignals zum zu bedienenden medizinischen Gerät. Die Steuerinformation wird dann unabhängig vom Vorliegen der Freigabeinformation zum zu bedienenden Gerät übertragen. Eine Steuereinheit des Gerätes prüft, ob gleichzeitig oder innerhalb des Zeitfensters, d. h. kurz vor oder kurz nach dem Empfang der Steuerinformation, das Freigabesignal gesendet worden ist. Dadurch können Fehlbedienungen einfach und wirkungsvoll vermieden werden.

Weitere Merkmale und Vorteile der Erfindung ergeben sich aus der folgenden Beschreibung, die in Verbindung mit den beigefügten Figuren die Erfindung anhand von Ausführungsbeispielen näher erläutert.

Es zeigen:
- Figur 1: eine Fernbedienung zum drahtlosen Steuern eines medizinischen Gerätes, die ein Smartphone und eine Dockingstation umfasst, gemäß einer ersten Ausführungsform der Erfindung;
- Figur 2: das Smartphone und die Dockingstation nach Figur 1 im getrennten Zustand;
- Figur 3: ein Blockschaltbild der Fernbedienung nach den Figuren 1 und 2 zum drahtlosen Steuern eines Operationstischs;
- Figur 4: ein Blockschaltbild einer ein Smartphone und eine Dockingstation umfassenden Fernbedienung zur drahtlosen Steuerung eines Operationstischs gemäß einer zweiten Ausführungsform der Erfindung;
- Figur 5: ein Blockschaltbild einer ein Smartphone und eine Dockingstation umfassenden Fernbedienung zur Steuerung eines Operationstischs gemäß einer dritten Ausführungsform der Erfindung;
- Figur 6: eine ein Smartphone und eine Dockingstation umfassende Fernbedienung gemäß einer vierten Ausführungsform der Erfindung; und
- Figur 7: das Smartphone und die Dockingstation nach Figur 6 in einem getrennten Zustand.

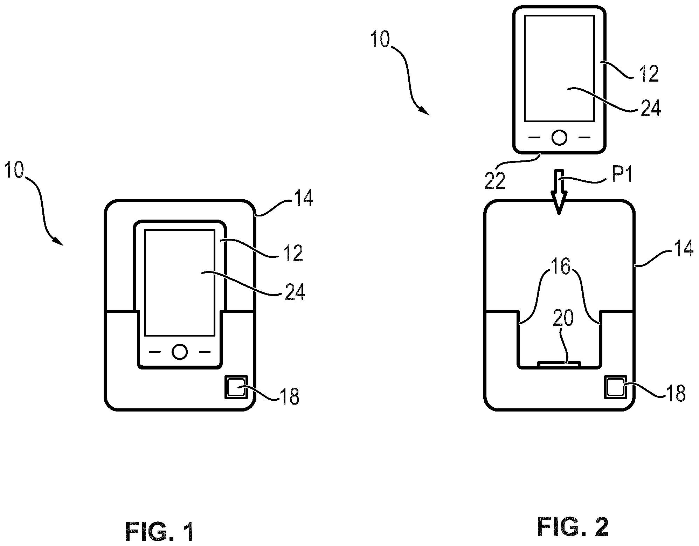

Figur 1 zeigt eine Fernbedienung 10, die ein Smartphone 12 und eine Dockingstation 14 umfasst. Das Smartphone 12 dient als erste Eingabeeinheit der Fernbedienung 10.

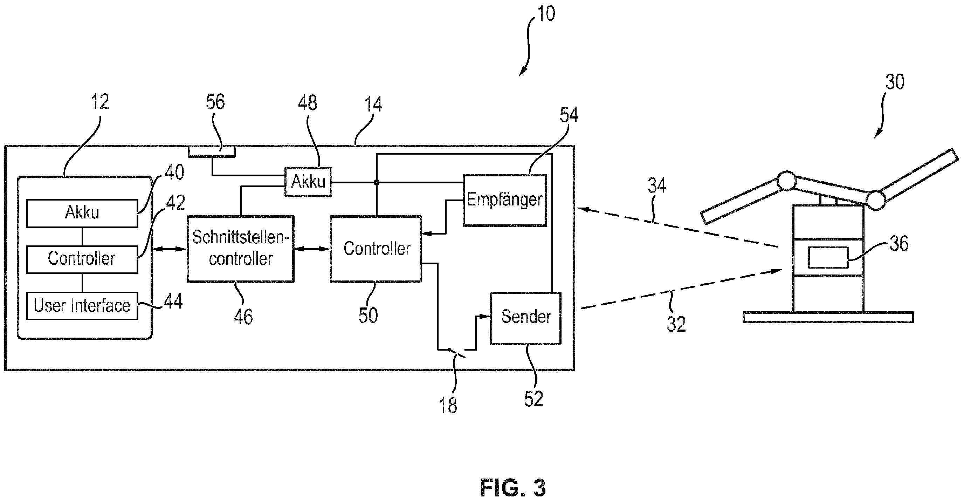

Die erste Eingabeeinheit 12 umfasst einen Touchscreen 24 zur Eingabe einer Steuerinformation zum Aktivieren und/oder Deaktivieren einer Steuerfunktion eines zu steuernden medizinischen Gerätes, im vorliegenden Ausführungsbeispiel zum Steuern eines in Figur 3 dargestellten Operationstischs 30. Die Dockingstation 14 umfasst eine als Taster 18 ausgebildete zweite Eingabeeinheit zur Eingabe einer Freigabeinformation. Beim ersten Ausführungsbeispiel der Erfindung erfolgt die Übertragung der über die erste Eingabeeinheit eingegebene Steuerinformation über die Dockingstation 14 zum zu steuernden medizinischen Gerät 30. Die Dockingstation 14 überträgt die Steuerinformation jedoch nur dann zum zu steuernden medizinischen Gerät 30, wenn gleichzeitig zur Eingabe der Steuerinformation über die erste Eingabeeinheit 12 die Freigabeinformation über den Taster 18 eingegeben wird, d. h. der Taster 18 muss bei der Eingabe der Steuerinformation über die erste Eingabeeinheit gedrückt sein.

Alternativ oder zusätzlich kann die Steuerinformation auch dann von der Dockingstation zum zu steuernden medizinischen Gerät übertragen werden, wenn die Freigabeinformation kurz vor oder kurz nach der Eingabe der Steuerinformation über die erste Eingabeeinheit erfolgt. Als Zeitspanne für den engen zeitlichen Zusammenhang der Eingabe der Freigabeinformation vor oder nach der Eingabe der Steuerinformation wird hierbei ein Zeitraum von plus/minus zwei Sekunden, vorzugsweise plus/minus einer Sekunde oder plus/minus 0,5 Sekunden angesehen. Hierdurch wird sichergestellt, dass die Eingabe der Steuerinformation und die Eingabe der Freigabeinformation im engen zeitlichen Zusammenhang stehen muss, damit die Steuerfunktion des Gerätes 30 ausgeführt wird.

Die Dockingstation 14 und das Smartphone 12 bilden zusammen die Fernbedienung 10. In Figur 2 sind Smartphone 12 und Dockingstation 14 in getrenntem Zustand gezeigt. Zum Verbinden des Smartphone mit der Dockingstation 14 wird das Smartphone in Richtung des Pfeils P1 in seitliche Aufnahmebereiche 16 der Dockingstation 14 hineingeschoben, bis ein Steckverbinder 20 der Dockingstation 14 mit einem komplementären Steckverbinder 22 des Smartphone 12 verbunden ist. Zur Eingabe der mindestens einen Steuerfunktion verfügt das Smartphone über einen Touchscreen 24, über den mindestens ein Eingabefeld zur Eingabe der Steuerinformation bereitgestellt wird. Alternativ oder zusätzlich kann der Touchscreen ein Texteingabefeld bereitstellen, über das ein Steuerbefehl zum Erzeugen der Steuerinformation eingebbar ist. Ferner können Anzeigefelder zur Anzeige durch die Fernbedienung 10 empfangenen Statusinformationen vorgesehen sein.

In Figur 3 ist ein Blockschaltbild der Fernbedienung 10 zusammen mit einer schematischen Darstellung des zu bedienenden Operationstischs 30 dargestellt. Das Smartphone 12 enthält zur Stromversorgung einen Akkumulator 40, einen Controller 42 und einen als Touchscreen 24 ausgeführtes User-Interface 44. Das Smartphone 12 ist über die Steckverbinder 20, 22 mit einem Schnittstellenkontroller 46 der Dockingstation 14 verbunden. Die Dockingstation 14 hat ferner einen Akkumulator 48, einen Dockingstations-Controller 50, eine Sendeeinheit 52 zum Senden von Steuerinformationen 32 einer Kommunikationseinheit 36 des Operationstisch und eine Empfängereinheit 54 zum Empfangen von Statusinformationen und/oder von Bestätigungsanforderungen der Kommunikationseinheit 36 des Operationstischs 30. Die Dockingstation 14 hat einen weiteren Steckverbinder 56, über den der Dockingstation 14 zumindest elektrische Energie zum Aufladen des Akkumulators 48 zuführbar ist. Der Akkumulator 40 des Smartphones 12 kann über eine durch den Schnittstellencontroller 46 bereitgestellte aus dem Akkumulator 48 gespeiste Stromversorgung oder alternativ direkt durch eine Stromversorgung über den Steckverbinder 56 aufgeladen werden. Der Schnittstellencontroller 46 überträgt insbesondere die über den Touchscreen des Smartphones 12 eingegebene Steuerinformation zum Dockingstationscontroller 50 und überträgt die vom Operationstisch 30 gesendeten, von der Empfängseinheit 54 empfangenen und vom Dockingcontroller 50 verarbeiteten Statusinformationen 34 des Operationstischs 30 zum Smartphone 12, das die Statusinformation über dem Touchscreen 24 ausgibt und/oder eine Auswahl von Steuerfunktionen abhängig von den Statusinformationen über den Touchscreen 24 ausgibt. Die Übertragung der zu sendenden Steuerinformation vom Kontroller 50 zum Sender 52 erfolgt nur bei geschlossenem Taster 18.

Bei alternativen Ausführungsformen kann der Taster 18 mit einem Eingang des Dockingstationscontrollers 50 verbunden sein, sodass dieser die Tastzustände des Tasters 18 erfasst und abhängig von den Tastzuständen zumindest ein Teil der über Touchscreen 24 eingegebenen Steuerinformationen nur dann zur Sendeeinheit 52 überträgt, wenn die Betätigung des Tasters 18 vom Dockingstationscontroller 50 detektiert worden ist. Vorzugsweise werden bei speziellen Ausführungsformen nur die Steuerinformationen in Abhängigkeit des Schaltzustandes 18 vom Dockingstationscontroller 50 zur Sendeeinheit 52 übertragen, die bei einer versehentlichen Aktivierung unmittelbar zu einer Beeinträchtigung der Behandlung des Patienten führen können.

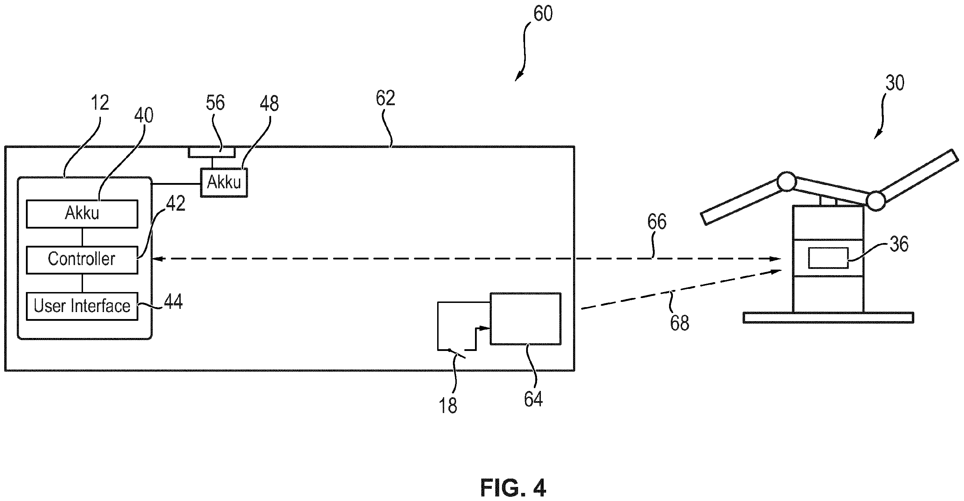

In Figur 4 ist ein Blockschaltbild einer Fernbedienung 60 mit einer Dockingstation 62 und dem Smartphone 12 gemäß einer zweiten Ausführungsform dargestellt. Elemente mit gleichem Aufbau oder gleicher Funktion haben die gleichen Bezugszeichen. Die Dockingstation 62 hat wie die Dockingstation 14 nach Figur 3 einen Akkumulator 48, der als zusätzliche Stromversorgung des Smartphones 12 und zum Aufladen des Akkumulators 40 des Smartphones 12 dient. Der Akkumulator 48 ist über den Steckverbinder 56 mit einer externen Stromversorgungseinheit (nicht dargestellt) verbindbar und kann über diese Steckverbindung 56 einfach aufgeladen werden. Im Unterschied zur Fernbedienung 10 gemäß der ersten Ausführungsform sendet das Smartphone 12 unabhängig vom Betätigungszustand des Tasters 18 eine Steuerinformation 66 zur Kommunikationseinheit 36 des zu steuernden Operationstischs 30. Die Datenübertragung zwischen der Kommunikationseinheit 36 und dem Smartphone 12 erfolgt bidirektional, d. h., dass auch Statusinformationen des Operationstischs 30 von der Kommunikationseinheit 36 zum Smartphone 12 übertragen werden.

Die Dockingstation 62hat ferner eine passive Sendeeinheit 64, die bei einer Stromversorgung durch das von einer als Schreib-/Leseeinheit ausgebildeten Kommunikationseinheit 36 des Operationstischs 30 in Abhängigkeit des Schaltzustandes des Tasters 18 eine Information zur Kommunikationseinheit 36 des Operationstischs 30 überträgt, die von der Kommunikationseinheit 36 als Freigabeinformation interpretiert wird. Die Kommunikationseinheit 36 und/oder eine weitere Steuereinheit des Operationstischs 30 führt die Steuerfunktion nur beim gleichzeitigen Vorliegen der Eingabeinformation der Freigabeinformation aus und/oder dann wenn der Empfang des Steuerinformation und der Freigabeinformation in engen zeitlichen Zusammenhang erfolgt. Was hierbei als enger zeitlicher Zusammenhang angesehen wird, ist bereits in Verbindung mit der ersten Ausführungsform der Erfindung erläutert worden. Die passive Sendeeinheit ist im vorliegenden Ausführungsbeispiel ein passiver Transponder. Bei anderen Ausführungsformen können auch andere, insbesondere aktive Sendeeinheiten eingesetzt werden.

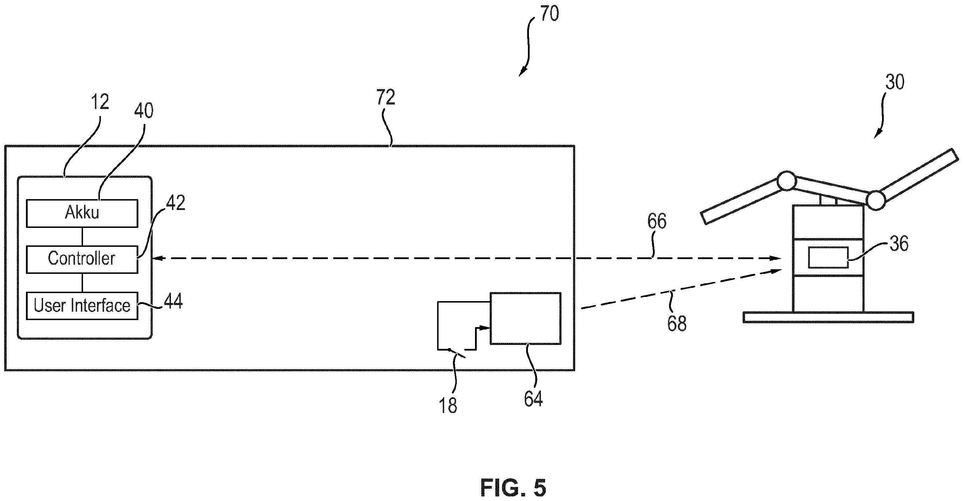

In Figur 5 ist Blockschaltbild einer Fernbedienung 70 mit einer Dockingstation 72 und dem Smartphone 12 gemäß einer dritten Ausführungsform gezeigt. Im Unterschied zur Fernbedienung 60 gemäß der zweiten Ausführungsform hat die Dockingstation 72 der Fernbedienung 70 der dritten Ausführungsform keinen Akkumulator. Die Funktion des Senders 64 zur Erzeugung und Übertragung der Freigabeinformation 68 und das Erzeugen der Steuerinformation 66 mit Hilfe des Smartphones 12 und die Übertragung der Steuerinformation zur Kommunikationseinheit 36 des Operationstischs 30 erfolgt in gleicher Weise wie im Zusammenhang mit der zweiten Ausführungsform der Erfindung erläutert. Der Steckverbinder 20 der Dockingstation 72 ist mit einem weiteren Steckverbinder 74 der Dockingstation 72 verbunden, sodass zumindest die Stromversorgung und vorzugsweise auch der Datenaustausch zwischen einer externen Steuereinheit und dem Smartphone 12 über die Steckverbinder 74, 20, 22 möglich ist.

Die Datenübertragung zwischen dem Smartphone 12 und der Kommunikationseinheit 36 kann in allen drei beschriebenen Ausführungsformen drahtlos erfolgen. Insbesondere erfolgt die Datenübertragung vom Smartphone 12 zur Kommunikationseinheit 36 des OP-Tischs 30 über eine der Kommunikationsarten WIFI, Bluetooth, Infrarot, Nahfeldkommunikationen und/oder Funksignalen. Dabei kann die Übertragung der Steuerinformation vom Smartphone 12 zur Kommunikationseinheit 36 auf eine erste Kommunikationsart erfolgen und die Übertragung von Informationen von der Kommunikationseinheit 36 zum Smartphone 12 auf eine andere Kommunikationsart.

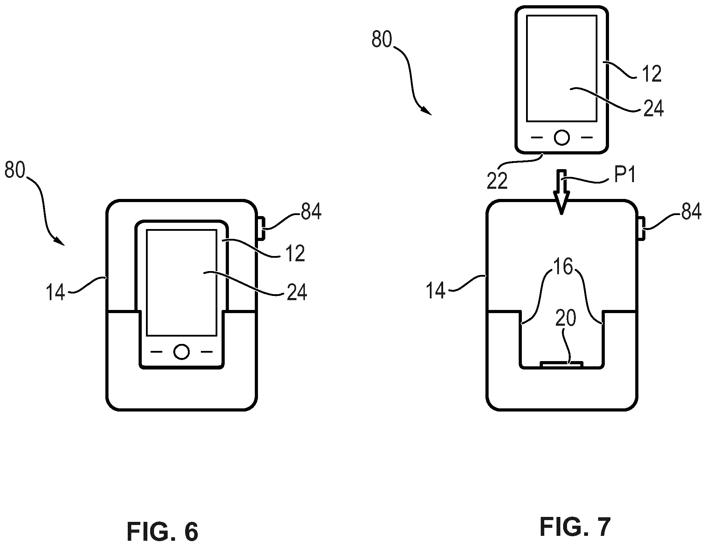

Figur 6 und 7 zeigen eine Fernbedienung 80 mit einer Dockingstation 82 und einem Smartphone 12 gemäß einer vierten Ausführungsform der Erfindung. Im Unterschied zur Fernbedienung 10 der ersten Ausführungsform der Erfindung weist die Dockingstation 82 an seiner rechten Außenseite im oberen Drittel ein als zweite Eingabeeinheit dienenden Taster 84 auf. Mit dieser Fernbedienung 80 sind die in den Figuren 3 bis 5 beschriebenen Steuerabläufe jeweils alternativ möglich, die in Verbindung mit der ersten, zweiten und dritten Ausführungsform beschrieben worden sind.

Als Dockingstation wird ein eine zweite Eingabeeinheit aufweisendes Gehäuse angesehen, dass mechanisch mit dem einen Touchscreen aufweisenden als erste Eingabeeinheit dienenden elektrischen Gerät mechanisch koppelbar ist. Besonders vorteilhaft ist es, wenn das in der Dockingstation aufgenommene Gerät von der Dockingstation an mindestens vier, vorzugsweise an fünf Seiten vollständig umschlossen ist. Besonders vorteilhaft ist es, wenn das Gehäuse der Dockingstation auch die sechste Seite des den Touchscreen aufweisenden elektrischen Gerätes bedeckt, wobei weiterhin Anzeigen auf dem Touchscreen durch die Abdeckungen durchsichtbar sind und der Touchscreen durch die Abdeckung hindurch bedienbar ist.

Die Dockingstation ist vorzugsweise als Cover, d. h. als Hülle, ausgebildet, in das das Smartphone 12 eingelegt wird. Die in den Blockschaltbildern der Figuren 3 bis 5 gezeigten weiteren Elemente sind als elektronische Schaltungen in das Cover integriert. Das Smartphone 12 wird in dieses Cover aufgenommen. Die elektrische Schaltung hat eine Schnittstelle zum Smartphone 12, die drahtgebunden oder drahtlos sein kann.

Als drahtgebundene Schnittstelle kann eine USB-Schnittstelle dienen, vorzugsweise über einen Mikro-USB-Connector. Als drahtlose Schnittstelle kann eine Bluetooth-Verbindung genutzt werden. Die Sendeeinheit 52 und die Empfangseinheit 54, die zur Kommunikation mit dem zu steuernden medizinischen Gerät 30 vorgesehen sind, basieren auf einer Infrarotübertragung und/oder einer Funkübertragung. Die Datenübertragung zwischen dem Controller 50 der Dockingstation 14 und dem Smartphone 12 erfolgt vorzugsweise bidirektional, sodass auch Statusmeldungen des medizinischen Gerätes an das Smartphone 12 zur Anzeige und/oder zur Weiterverarbeitung übertragen werden.

Alternativ zu den in den Figuren 1 bis 7 beschriebenen Smartphone 12 können auch andere einen Touchscreen aufweisenden elektrische Geräte eingesetzt werden, wie beispielsweise Medienabspielgeräte, Tablet-Computer, insbesondere ein iPad oder ein iPod der Firma Apple Computers Incorporation.

### Bezugszeichenliste

- 10, 60, 70, 80: Fernbedienung
- 12: Smartphone
- 14, 62, 72, 82: Dockingstation
- 16: Aufnahmebereiche
- 18, 84: Taster
- 20, 22: Steckverbinder
- 24: Touchscreen
- 30: Operationstisch
- 32, 66: Steuerinformation
- 34: Statusinformation
- 36: Kommunikationseinheit
- 40, 48: Akkumulator
- 42: Controller
- 44: User-Interface
- 46: Schnittstellencontroller
- 50: Dockingstationscontroller
- 52: Sendeeinheit
- 54: Empfangseinheit
- 56: Steckverbinder
- 64: Sender
- 68: Freigabeinformation

## Patentansprüche

1. Verfahren zum drahtlosen Steuern eines medizinischen Gerätes (30) mit Hilfe einer Fernbedienung (10, 60, 70, 80),
bei dem über mindestens eine über einen Touchscreen (24) der Fernbedienung (10, 60, 70, 80) bereitgestellte erste Eingabeeinheit (12) mindestens eine Steuerinformation zum Aktivieren und/oder Deaktivieren einer Steuerfunktion zum Steuern des Gerätes (30) eingegeben wird,
über eine von der ersten Eingabeeinheit (12) unabhängigen zweiten Eingabeeinheit (14, 62, 72, 82) der Fernbedienung (10, 60, 70, 80) mindestens eine Freigabeinformation eingegeben wird, und
bei dem bei einer Eingabe der Steuerinformation die Steuerfunktion des Geräts (30) nur aktiviert und/oder deaktiviert wird, wenn über die zweite Eingabeeinheit (14, 62, 72, 82) die Freigabeinformation eingegeben wird,
**dadurch gekennzeichnet, dass**
die zweite Eingabeeinheit (14, 62, 72, 82) durch mindestens einen Schalttransponder bereitgestellt wird, der einen Transponder (64) und ein mit diesem verbundenes Betätigungselement (18, 84) umfasst, und
das zu steuernde medizinische Gerät ein OP-Tisch (30) ist.

2. Verfahren nach Anspruch 1,
**dadurch gekennzeichnet, dass** die Steuerinformation nur von der Fernbedienung (10, 60, 70, 80) zum Gerät (30) gesendet wird, wenn die Steuerinformation über die erste Eingabeeinheit (12) und die Freigabeinformation über die zweite Eingabeeinheit (14, 62, 72, 82) gleichzeitig oder innerhalb eines voreingestellten Zeitfensters eingegeben werden.

3. Verfahren nach einem der vorhergehenden Ansprüche,
**dadurch gekennzeichnet, dass**
Daten mit der Steuerinformation von einer ersten Steuereinheit der ersten Eingabeeinheit (12) zu einer zweiten Steuereinheit der zweiten Eingabeeinheit (14, 62, 72, 82) übertragen werden, und dass die Steuerinformation von der zweiten Steuereinheit nur dann weiter zum Gerät (30) übertragen wird, wenn durch die zweite Steuereinheit die Freigabeinformation gleichzeitig oder innerhalb eines voreingestellten Zeitfensters detektiert wird.

4. Verfahren nach Anspruch 1,
**dadurch gekennzeichnet, dass**
die Steuerfunktion des Gerätes (30) durch eine Steuereinheit (36) des Gerätes nur dann aktiviert und/oder deaktiviert wird, wenn die Steuerinformation und die Freigebeinformation innerhalb eines voreingestellten Zeitfensters oder gleichzeitig von der Fernbedienung (10, 60, 70, 80) zum Gerät übertragen werden.

5. Verfahren nach einem der vorhergehenden Ansprüche,
**dadurch gekennzeichnet, dass**
die erste Eingabeeinheit durch ein Smartphone (12), einen Tablet-Computer und/oder ein tragbares digitales Medienabspielgerät bereitgestellt wird.

6. Verfahren nach einem der vorhergehenden Ansprüche,
**dadurch gekennzeichnet, dass**
der Schalttransponder zumindest in einem Schaltzustand des Betätigungselements (18, 84) mit einer Kommunikationseinheit (36), vorzugsweise mit einer Leseeinheit, kommuniziert, wobei die Kommunikationseinheit (36) mit einer Steuereinheit des Gerätes zumindest über eine Datenleitung verbunden ist.

7. Verfahren nach einem der vorhergehenden Ansprüche,
**dadurch gekennzeichnet, dass**
die zweite Eingabeeinheit (14, 62, 72, 82) ein Gehäuse mit einem Aufnahmefach hat, in dem die erste Eingabeeinheit (12) derart aufgenommen wird, dass der Touchscreen (24) der ersten Eingabeeinheit (12) bedienbar, vorzugsweise frei zugänglich ist.

8. Verfahren nach Anspruch 7,
**dadurch gekennzeichnet, dass**
die erste Eingabeeinheit (12) in das Aufnahmefach eingeschoben wird.

9. Verfahren nach Anspruch 7 oder 8,
**dadurch gekennzeichnet, dass**
die erste Eingabeeinheit (12) über mindestens einen Steckverbinder (20, 22) mit dem Gehäuse verbunden wird, wobei über den Steckverbinder (20, 22) eine Stromversorgung der ersten Eingabeeinheit (12) erfolgt und/oder Daten zwischen einer Steuereinheit (42) der ersten Eingabeeinheit (12) und einer im Gehäuse angeordneten zweiten Steuereinheit (50) übertragen werden.

10. Verfahren nach einem der Ansprüche 7 bis 9,
**dadurch gekennzeichnet, dass**
die Freigabeinformation über ein von der Außenseite des Gehäuses zugängliches außerhalb des Aufnahmebereichs angeordnetes Betätigungselement (14, 84) der zweiten Eingabeeinheit (14, 62, 72, 82), eingegeben wird.

11. Verfahren nach einem der vorhergehenden Ansprüche,
**dadurch gekennzeichnet, dass**
mit Hilfe der Steuerfunktion einen Antrieb des zu steuernden medizinischen Gerätes (30) aktiviert und/oder deaktiviert wird, insbesondere einen Stellantrieb und/oder einen Antrieb für ein Verriegelungselement.

12. Verfahren nach einem der vorhergehenden Ansprüche,
**dadurch gekennzeichnet, dass**
Daten mit der Steuerinformation und/oder mit der Freigabeinformation von der ersten Eingabeeinheit (12), von der zweiten Eingabeeinheit (14, 62, 72, 82) oder von einer zweiten Steuereinheit der zweiten Eingabeeinheit verschlüsselt zum Gerät (30) übertragenen werden.

13. Vorrichtung zur drahtlosen Steuerung eines medizinischen Gerätes (30),
mit einem Gehäuse zur Aufnahme eines separat nutzbaren Gerätes (12), das mindestens eine über einen Touchscreen (24) bereitgestellte Eingabeeinheit in Form eines separaten nutzbaren Gerätes (12) zur Eingabe mindestens einer Steuerinformation zum Aktivieren und/oder Deaktivieren einer Steuerinformation zum Steuern des zu steuernden medizinischen Gerätes (30) hat,
wobei das Gehäuse eine von der ersten Eingabeeinheit unabhängige zweite Eingabeeinheit (14, 62, 72, 82) zum Eingeben mindestens einer Freigabeinformation enthält, wobei bei einer Eingabe der Steuerinformation die Steuerfunktion des Geräts (30) nur aktiviert und/oder deaktiviert wird, wenn über die zweite Eingabeeinheit (14, 62, 72, 82) die Freigabeinformation eingegeben wird,
**dadurch gekennzeichnet, dass**
die zweite Eingabeeinheit (14, 62, 72, 82) durch mindestens einen Schalttransponder bereitgestellt wird, der einen Transponder (64) und ein mit diesem verbundenes Betätigungselement (18, 84) umfasst, und
das zu steuernde medizinische Gerät ein OP-Tisch (30) ist.

## Claims

1. A method for wirelessly controlling a medical device (30) by means of a remote control (10, 60, 70, 80),
in which at least one control information for activating and/or deactivating a control function for controlling the device (30) is input via at least one first input unit (12) provided via a touchscreen (24) of the remote control (10, 60, 70, 80),
at least one release information is input via a second input unit (14, 62, 72, 82) of the remote control (10, 60, 70, 80), which is independent of the first input unit (12),
and
in which the control function of the device (30) is only activated and/or deactivated when the control information is input if the release information is input via the second input unit (14, 62, 72, 82),
**characterized in that**
the second input unit (14, 62, 72, 82) is provided by at least one switching transponder, which comprises a transponder (64) and an actuating element (18, 84) connected thereto, and
the medical device to be controlled is an operating table (30).

2. The method according to claim 1,
**characterized in that** the control information is only sent to the device (30) by the remote control (10, 60, 70, 80) if the control information via the first input unit (12) and the release information via the second input unit (14, 62, 72, 82) are input simultaneously or within a preset time window.

3. The method according to any one of the preceding claims,
**characterized in that**
data is transmitted with the control information from a first control unit of the first input unit (12) to a second control unit of the second input unit (14, 62, 72, 82), and that the control information is only transmitted further from the second control unit to the device (30) if the second control unit detects the release information at the same time or within a preset time window.

4. The method according to claim 1,
**characterized in that**
the control function of the device (30) is only activated and/or deactivated by a control unit (36) of the device if the control information and the release information are transmitted within a preset time window or simultaneously from the remote control (10, 60, 70, 80) to the device.

5. The method according to any one of the preceding claims,
**characterized in that**
the first input unit is provided by a smartphone (12), a tablet computer and/or a wearable digital media player.

6. The method according to any one of the preceding claims,
**characterized in that**
the switching transponder communicates with a communication unit (36), preferably a reading unit, in at least one switching state of the actuating element (18, 84), wherein the communication unit (36) is connected to a control unit of the device via at least one data line.

7. The method according to any one of the preceding claims,
**characterized in that**
the second input unit (14, 62, 72, 82) comprises a housing with a receiving compartment in which the first input unit (12) is housed in such a manner that the touchscreen (24) of the first input unit (12) is operable, preferably freely accessible.

8. The method according to claim 7,
**characterized in that**
the first input unit (12) is slid into the receiving compartment.

9. The method according to claim 7 or 8,
**characterized in that**
the first input unit (12) is connected to the housing via at least one plug-in connector (20, 22), wherein power is supplied to the first input unit (12) and/or data is transmitted between a control unit (42) of the first input unit (12) and a second control unit (50) disposed in the housing via the plug-in connector (20, 22).

10. The method according to any one of claims 7 to 9,
**characterized in that**
the release information is input via an actuating element (14, 84) of the second input unit (14, 62, 72, 82) accessible from the outside of the housing and disposed outside the receiving area.

11. The method according to any one of the preceding claims,
**characterized in that**
a drive of the medical device (30) to be controlled, particularly an actuator and/or a drive for a locking element, is activated and/or deactivated by means of the control function.

12. The method according to any one of the preceding claims,
**characterized in that**
data including the control information and/or the release information from the first input unit (12), from the second input unit (14, 62, 72, 82), or from a second control unit of the second input unit is transmitted to the device (30) in encrypted form.

13. An apparatus for wirelessly controlling a medical device (30),
having a housing for receiving a separately usable device (12), which at least comprises an input unit provided via a touchscreen (24) in the form of a separately usable device (12) for inputting at least one control information for activating and/or deactivating a control information for controlling the medical device (30) to be controlled,
wherein the housing contains a second input unit (14, 62, 72, 82) independent of the first input unit for inputting at least one release information, wherein the control function of the device (30) is only activated and/or deactivated when the control information is input if the release information is input via the second input unit (14, 62, 72, 82),
**characterized in that**
the second input unit (14, 62, 72, 82) is provided by at least one switching transponder, which comprises a transponder (64) and an actuating element (18, 84) connected thereto, and
the medical device to be controlled is an operating table (30).

## Revendications

1. Procédé pour la commande sans fil d'un appareil médical (30) à l'aide d'une commande à distance (10, 60, 70, 80),
dans lequel par le biais d'au moins une première unité de saisie (12) fournie par le biais d'un écran tactile (24) de la commande à distance (10, 60, 70, 80) au moins une information de commande pour l'activation et/ou la désactivation d'une fonction de commande pour la commande de l'appareil (30) est saisie,
au moins une information d'autorisation est saisie par le biais d'une seconde unité de saisie (14, 62, 72, 82) de la commande à distance (10, 60, 70, 80) indépendante de la première unité de saisie (12), et
dans lequel lors d'une saisie de l'information de commande la fonction de commande de l'appareil (30) n'est activée et/ou désactivée que lorsque l'information d'autorisation est saisie par le biais de la seconde unité de saisie (14, 62, 72, 82),
**caractérisé en ce que**
la seconde unité de saisie (14, 62, 72, 82) est fournie par le biais d'au moins un transpondeur de commutation qui comprend un transpondeur (64) et un élément d'actionnement (18, 84) relié à ce dernier, et
l'appareil médical à commander est une table chirurgicale (30) .

2. Procédé selon la revendication 1,
**caractérisé en ce que** l'information de commande n'est envoyée par la commande à distance (10, 60, 70, 80) à l'appareil (30) que lorsque l'information de commande est émise par le biais de la première unité de saisie (12) et l'information d'autorisation est saisie par le biais de la seconde unité de saisie (14, 62, 72, 82) en même temps ou au sein d'un créneau prédéfini.

3. Procédé selon l'une des revendications précédentes,
**caractérisé en ce que**
des données avec l'information de commande sont transmises par une première unité de commande de la première unité de saisie (12) à une seconde unité de commande de la seconde unité de saisie (14, 62, 72, 82), et **en ce que** l'information de commande n'est ensuite transmise à l'appareil (30) par la seconde unité de commande que lorsque l'information d'autorisation est détectée en même temps ou au sein d'un créneau prédéfini par le biais de la seconde unité de commande.

4. Procédé selon la revendication 1,
**caractérisé en ce que**
la fonction de commande de l'appareil (30) n'est ensuite activée et/ou désactivée par le biais d'une unité de commande (36) de l'appareil que lorsque l'information de commande et l'information d'autorisation sont transmises au sein d'un créneau prédéfini ou en même temps par la commande à distance (10, 60, 70, 80) à l'appareil.

5. Procédé selon l'une des revendications précédentes,
**caractérisé en ce que**
la première unité de saisie est fournie par le biais d'un smartphone (12), d'une tablette et/ou d'un appareil de lecture multimédia numérique portable.

6. Procédé selon l'une des revendications précédentes,
**caractérisé en ce que**
le transpondeur de commutation communique dans un état de commutation de l'élément d'actionnement (18, 84) avec une unité de communication (36), de préférence avec une unité de lecture, dans lequel l'unité de communication (36) est reliée à une unité de commande de l'appareil au moins par le biais d'un câble de données.

7. Procédé selon l'une des revendications précédentes,
**caractérisé en ce que**
la seconde unité de saisie (14, 62, 72, 82) a un boîtier avec un réceptacle dans lequel la première unité de saisie (12) est reçue, **en ce que** l'écran tactile (24) de la première unité de saisie (12) est utilisable, de préférence en accès libre.

8. Procédé selon la revendication 7,
**caractérisé en ce que**
la première unité de saisie (12) est introduite dans le réceptacle.

9. Procédé selon la revendication 7 ou 8,
**caractérisé en ce que**
la première unité de saisie (12) est reliée par le biais d'au moins un connecteur enfichable (20, 22) au boîtier, dans lequel une alimentation électrique de la première unité de saisie (12) s'opère par le biais du connecteur enfichable (20, 22) et/ou des données sont transmises entre une unité de commande (42) de la première unité de saisie (12) et une seconde unité de commande (50) disposée dans le boîtier.

10. Procédé selon l'une des revendications 7 à 9,
**caractérisé en ce que**
l'information d'autorisation est saisie par le biais d'un élément d'actionnement (14, 84) de la seconde unité de saisie (14, 62, 72, 82), élément d'actionnement qui est accessible par le côté externe du boîtier et disposé à l'extérieur de la zone de réception.

11. Procédé selon l'une des revendications précédentes,
**caractérisé en ce que**
à l'aide de la fonction de commande un mécanisme d'entraînement de l'appareil médical (30) à commander est activé et/ou désactivé, en particulier un mécanisme de commande et/ou un mécanisme d'entraînement pour un élément de verrouillage.

12. Procédé selon l'une des revendications précédentes,
**caractérisé en ce que**
des données avec l'information de commande et/ou avec l'information d'autorisation sont transmises sous forme codée par la première unité de saisie (12), par la seconde unité de saisie (14, 62, 72, 82) ou par une seconde unité de commande de la seconde unité de saisie à l'appareil (30).

13. Dispositif pour la commande sans fil d'un appareil médical (30),
avec un boîtier pour la réception d'un appareil utilisable séparément (12), qui a au moins une unité de saisie fournie par le biais d'un écran tactile (24) sous la forme d'un appareil utilisable séparément (12) pour la saisie d'au moins une information de commande pour l'activation et/ou la désactivation d'une information de commande pour la commande de l'appareil médical (30) à commander,
dans lequel le boîtier contient une seconde unité de saisie (14, 62, 72, 82) indépendante de la première unité de saisie pour la saisie d'au moins une information d'autorisation, dans lequel lors d'une saisie de l'information de commande la fonction de commande de l'appareil (30) n'est activée et/ou désactivée que lorsque l'information d'autorisation est saisie par le biais de la seconde unité de saisie (14, 62, 72, 82),
**caractérisé en ce que**
la seconde unité de saisie (14, 62, 72, 82) est fournie par le biais d'au moins un transpondeur de commutation qui comprend un transpondeur (64) et un élément d'actionnement (18, 84) relié à ce dernier, et
l'appareil médical à commander est une table chirurgicale (30) .
